Europäisches Patentamt

European Patent Office

Office européen des brevets

(19)

(11) Veröffentlichungsnummer: 0 401 579 A1

# (12) EUROPÄISCHE PATENTANMELDUNG

(21) Anmeldenummer: 90109518.2

(51) Int. Cl.5: A61B 19/02, A47B 31/00

(22) Anmeldetag: 19.05.90

(30) Priorität: 03.06.89 DE 3918162

(43) Veröffentlichungstag der Anmeldung:
12.12.90 Patentblatt 90/50

(84) Benannte Vertragsstaaten:
BE DE FR GB IT NL

(71) Anmelder: RIWOPLAN MEDIZIN-TECHNISCHE
EINRICHTUNGSGESELLSCHAFT MBH
Pforzheimer Strasse 24
D-7134 Knittlingen(DE)

(72) Erfinder: Mönch, Harry
Schwabstrasse 4
D-7134 Knittlingen(DE)
Erfinder: Weisert, Willi
Mühlweg 11
D-7519 Oberderdingen(DE)

(74) Vertreter: Wilcken, Thomas, Dipl.-Ing. et al
Musterbahn 1
D-2400 Lübeck(DE)

(54) Mobiler Gerätetisch.

(57) Der beschriebene mobile Gerätetisch (1,2,4,5), insbesondere zum Transport und/oder zur Aufnahme von medizinischen Instrumenten, Geräten oder dergleichen, kann durch Verwendung normierter Bauteile wie Arbeitsaufnahmeplatten (11), Schubladenblöcke (18) oder dergleichen zweckangepaßt konfektioniert werden. Dabei sind sämtliche Bauteile zur Vermeidung von Keimverschleppungen durch zu den Bauteilen komplementär ausgebildete Kunststofformteile (7,9,14,15, 20,21) ummantelt, so daß die Metallteile vor Berührung mit aggressiven und daher korrosionsverursachenden Medien geschützt sind.

FIG. 1

FIG. 2

EP 0 401 579 A1

Die Erfindung betrifft einen mobilen Gerätetisch, insbesondere zum Transport und/oder zur Aufnahme von medizinischen Instrumenten, Geräten oder dergleichen,umfassend ein auf Rollen ruhendes Traggestell , welches durch Verwendung normierter Bauteile wie Arbeitsaufnahmeplatten, Schubladenblöcken für eine oder mehrere Schubladen oder dergleichen dem Zweck angepaßt konfektionierbar ist.

Solche Gerätetische sind in vielfältigen Gestaltungen bekannt, wobei sie hinsichtlich ihrer äußeren Raumform, Einteilung und Ausführung von Ablageflächen, Schubkästen und dergleichen dem jeweiligen Verwendungszweck angepaßt sind. Dabei sind zur Vermeidung von unbeabsichtigten Keimverschleppungen die Oberflächen derartiger,in der klinischen Anwendung befindlicher Gerätetische mit einem galvanischen oder mit einem Farbüberzug oder auch mit einer Kunststoffbeschichtung versehen. Die mechanische Stabilität, die im täglichen Gebrauch gewährleistet sein muß, wird in der Regel durch Verschweißen der einzelnen Elemente, insbesondere der tragenden Teile wie Stützen, Streben oder dergleichen, erzielt, so daß die Grundkörper eine unlösbare Baueinheit bilden. Verschraubt werden lediglich Zusatz oder Einzelteile wie Gleitschienen, Türen oder dergleichen.

Der dem Stand der Technik anhaftende Nachteil besteht nun darin, daß die Gerätetische im allgemeinen unmittelbar in Anpassung an den je-weiligen Verwendungszweck entwickelt und gefer-tigt werden, so daß einzelne Elemente nur in sehr beschränktem Maße auf andere Bauformen übertragbar sind, woraus eine hohe Zahl von voneinander abweichenden Einzelteilen resultiert, was letztendlich zu einem sehr hohen Kostenanteil für die Entwicklung und Fertigung führt.

Ein weiterer Nachteil des Standes der Technik besteht in der teilweise ungenügenden Oberflächenbeschaffenheit, so daß Korrosionsschäden, die durch aggressive Reinigungs-und/oder Desinfektionsmittel, der Luftfeuchtigkeit sowie aufgrund von verschétteten Flüssigkeiten verursacht werden, zu einer nachhaltigen Beeinträchtigung der hygienischen Anforderungen führen mit der Folge unbeabsichtigter Keimverschleppungen und daraus für den Patienten sich ergebender Risiken.

Der Erfindung liegt daher die Aufgabe zugrunde, einen mobilen Gerätetisch vorzuschlagen, der neben leichter Konfektionierbarkeit in Anpassung an den jeweils vorgesehenen Verwendungszweck eine Oberflächenbeschaffenheit und -Gestaltung aufweist, daß der Gefahr einer Keimverschleppung weitestgehend entgegengewirkt wird.

Diese Aufgabe wird erfindungsgemäß dadurch gelöst, daß insbesondere die tragenden Bauteile aus an dem Traggestell befestigbaren Metallteilen bestehen, die sàmtlich durch komplementär ausgebildete, die Metallteile umschließende Kunststofformteile ummantelt sind.

Die damit erzielbaren Vorteile bestehen insbesondere darin, daß der unmittelbare Kontakt zwischen einem flüssigen Medium,wie beispielsweise einem Desinfektionsmittel,und dem korrosionsgefährdeten Metallteil unterbunden wird. Neben diesem Vorteil besteht aufgrund der Verwendung normierter Bauteile im Falle einer Beschädigung oder einer durch Verschleiß beeinträchtigten Gebrauchstüchtigkeit die Möglichkeit, daß Einzelteile problemlos ausgetauscht werden können.

Nach einem bevorzugten Ausführungsbeispiel kann das Traggestell zwei Grundkörper-Seitenteile aus zwei senkrecht aufragenden Tragsäulen umfassen, die lösbar miteinander verbunden sind durch eine gleichzeitig als Handhabe dienende obere Traverse und eine mit Rollen im Bereich ihrer beiden Enden bestückte untere Traverse. Dabei können die Tragsäulen mit in bestimmtem Raster angeordneten Aufnahmen für die Befestigung der genannten Bauteile versehen sein. Die die Tragsäulen ummantelnden Kunststofformteile können im Anschlußbereich an die die Rollen tragende Traverse von einer dichtend abschließenden Manschette umgeben sein. Anderen Endes können die die Tragsäulen ummantelnden Kunststofformteile durch das die als Handhabe ausgebildete Traverse überdek-kende Kunststofformteil übergriffen werden.

Nach einer weiteren Ausführungsform kann jede Arbeitsaufnahmeplatte eine metallene Verstärkungsplatte umfassen, die an ihrer Unterseite in Längsrichtung durch Traversen versteift ist, wobei die Verstärkungsplatte zwischen einem einer Arbeitsplatte und einem an die Form derselben und die der Traversen angepaßten Kunststofformteil abgedichtet eingebettet ist.

Bei einer Ausstattung mit Schubladen können die diese aufnehmenden Schubladenblöcke aus einem inneren und einem äußeren Kunststofformteil bestehen, zwischen zwelchen Versteifungselemente und/oder Versteifungsplatten angeordnet sein können. Es können aber auch sowohl die Schubläden als auch der Schubladenblock aus Kunststoff gefertigt sein.

Der erfindungsgemäße Gerätetisch ist nachstehend anhand eines in der Zeichnung dargestellten Ausführungsbeispieles näher erläutert. Es zeigt:

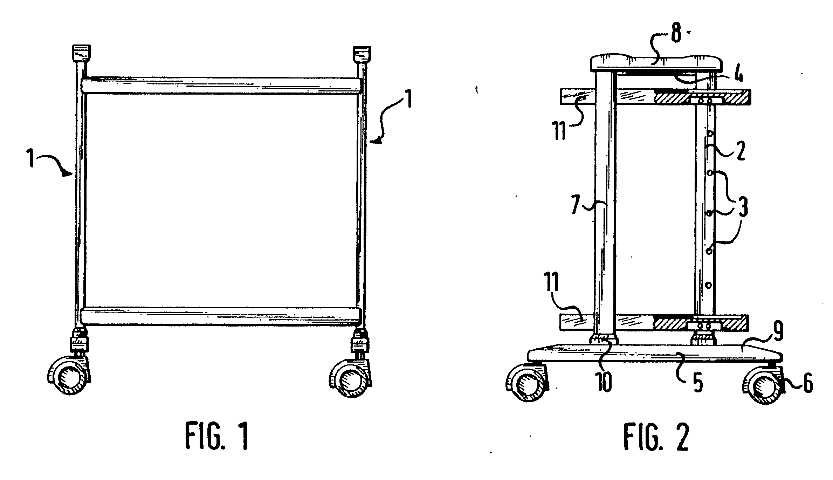

Figur 1 eine Frontansicht des Grundkörpers in der einfachsten Ausführung,

Figur 2 eine Seitenansicht des Grundkörpers nach Figur 1 in teilweise geschnittener Darstellung,

Figur 3 eine ausschnittweise Darstellung des Aufbaues der Arbeitsaufnahmeplatte nach Figur 2 vergrößert wiedergegeben,

Figur 4 die Verbindung einer Tragsäule mit der unteren Traverse im Teilauschnitt vergrößert dargestellt,

Figur 5 eine erste Kombinationsmöglichkeit mit zwei Arbeitsaufnahmeplatten,

Figur 6 eine zweite Kombinationsmöglichkeit mit zwei Arbeitsaufnahmeplatten und einem zwischen diesen angeordneten Schubladenblock,

Figur 7 eine dritte Kombinationsmöglichkeit mit einer ersten Arbeitsaufnahmeplatte, einem Schubladenblock und einer zweiten Arbeitsaufnahmeplatte jeweils im Abstand zueinander,

Figur 8 eine vierte Kombinationsmöglichkeit mit zwei im Abstand zueinander angeordneten Arbeitsaufnahmeplatten und zwischen diesen angeordneten Trägerelementen zur Aufnahme von beispielsweise Desinfektionsschalen,

Figur 9 eine fünfte Kombinationsmöglichkeit mit einer Arbeitsaufnahmeplatte und zwei Trägerelementenzur Aufnahme von zwei großen Desinfektionsbehältern,

Figur 10 eine sechste Kombinationsmöglichkeit mit einer Arbeitsaufnahmeplatte und zwei Trägerelementen teilen zur Aufnahme eines einzelnen, großvolumigen Desinfektionsbehälters.

Der erfindungsgemäße mobile Gerätetisch besteht im wesentlichen aus zwei in seitlichem Abstand zueinander angeordneten Grundkörperseitenteilen 1. Diese wiederum bestehen aus zwei senkrecht und parallel zueinander angeordneten Tragsäulen 2, die in bestimmtem Raster angeordnete Aufnahmen 3 aufweisen, beispielsweise in Form von Schraubgewinden. Die beiden Tragsäulen 2 sind durch eine gleichzeitig als Handhabe dienende obere Traverse 4 und eine untere Traverse 5 miteinander verbunden, die jeweils an ihren beiden diametral gegenüberliegenden Enden mit Rollen 6 zum Verfahren des Gerätetischs versehen sind. Die Verbindungen der Tragsäulen 2 mit den Traversen 4 und 5 sind lösbar ausgeführt, wobei die Demontage dieser Teile nur unter Anwendung von Werkzeugen möglich ist.

Die Bauteile 2, 4 und 5 sind aus Gründen der Stabilität aus Metall gefertigt und im Hinblick auf die hygienischen Anforderungen mit komplementär ausgebildeten Kunststofformteilen 7, 8, 9 ummantelt, die sich gegenseitig soweit übergreifen, daß ein unmittelbarer Kontakt der Metallteile mit flüssigen Medien wirksam unterbunden wird. Um das Eindringen von Flüssigkeiten in den Verbindungsbereich zwischen den unteren Enden der Tragsäulen 2 und der unteren Traverse 5 wirksam zu verhindern, weist das die Tragsäule 2 umgebende Kunststofformteil 7 in diesem Verbindungsbereich eine Manschette 10 auf, die sich dichtend an das die untere Traverse umgebende Kunststofformteil 9 anschließt.

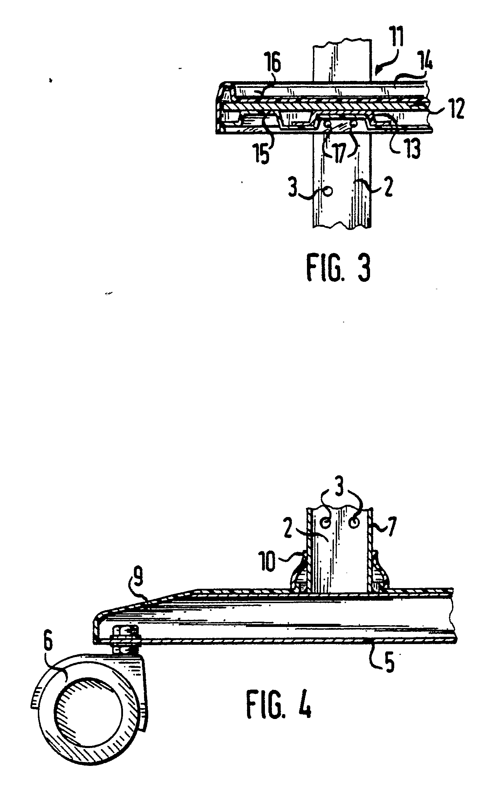

Die derartig konzipierten Grundkörper-Seitenteile 1 bilden die Seitenstützen für je nach Verwendungszweck des Gerätetisches ausgewählte Bauteile, wobei die Verbindung derselben mit den Seitenteilen 1 in den Aufnahmen 3 der Tragsäulen 2, im einfachsten Fall durch Verschraubung erfolgt. Zu diesen Bauteilen gehört eine Arbeitsaufnahmeplatte 11, die gemäß Figur 3 eine Verstärkungsplatte 12 umfaßt, die an ihrer Unterseite in Längsrichtung durch Traversen 13 versteift und in Kunststofformteile 14 und 15 eingebettet ist. Dabei umgreift das Kunststofformteil 14 die Stirnseiten der Verstärkungsplatte 12 und bildet eine Arbeitsplatte 16, während das Kunststofformteil 15 an die Unterseite der Verstärkungsplatte 12 und der Traversen 13 angepaßt und an den Rändern mit dem Kunststofformteil 14 verschweißt ist. Die Befestigung der Arbeitsaufnahmeplatte 11 an den Tragsäulen 2 erfolgt über Bohrungen 17.

Die verwendbaren Bauteile umfassen weiter einen Schubladenblock 18 zur Aufnahme von Schubkästen 19, welche Teile in Anlehnung an den zuvor erläuterten Aufbau gleichfalls sandwichartig aufgebaut sein können. Der Schubladenblock 18 weist in diesem Sinne ein äußeres Kunststofformteil 20 und ein inneres Kunststofformteil 21 auf, zwischen welchen Formteilen sich zur Verbesserung der mechanischen Stabilität Versteifungselemente und Versteifungsplatten befinden können. Die Schubkästen 19 laufen entsprechend dem Stand der Technik auf nicht gezeigten Teleskopschienen. Die Befestigung des Schubladenblockes 18 an den Tragsäulen 2 erfolgt in der gleichen, zuvor beschriebenen Art und Weise. Auch die Kästen 19 können mit Versteifungselementen versehen sein.

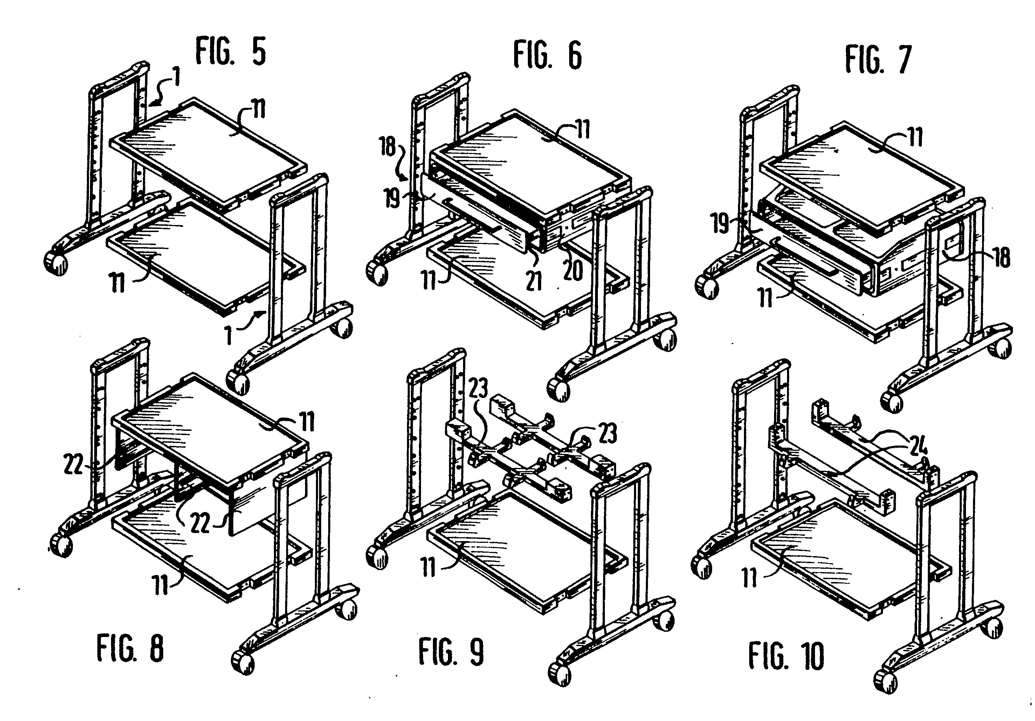

Bei der für die Aufnahme von Desinfektionsschalen vorgesehenen Ausführung entsprechend Figuren 8, 9 und 10 können die Arbeitsaufnahmeplatten 11 oder auch die Tragsäulen 2 direkt mit den entsprechenden Trägerelementen 22, 23, 24 ausgerüstet werden. Auch diese Trägerelemente besitzen zur Aufnahme der Gewichtskräfte jeweils einen nicht dargestellten metallischen Grundkörper, der von einem komplementär dazu ausgebildeten Kunststofformteil umgeben ist. Dabei können die die Trägerelemente 23 und 24 umgreifenden Kunststofformteile an ihrer Unterseite offen sein, während die Trägerelemente 22 durch Kunststofformteile vollständig umgeben sind.

Die die Tragsäulen 2 ummantelnden Kunststofformteile 7 sind so ausgeführt, daß alle Aufnahmen 3 überdeckt werden. Zur Befestigung der Bauteile sind daher die Kunststofformteile 7 lediglich an der Stelle mit Durchbrüchen zu versehen, hinter der sich die entsprechende Aufnahme 3 verbirgt.

In weiterer Ausbildung des erfindungsgemäßen Gerätetisches ist es denkbar, die Kunststofformteile in Art eines Schrumpfschlauches auf dem jeweiligen Metallgrundkörper festzulegen.

## Ansprüche

1. Mobiler Gerätetisch (1,2), insbesondere zum Transport und/oder zur Aufnahme von medizinischen Instrumenten, Geräten oder dergleichen, umfassend ein auf Rollen (6) ruhendes Traggestell (2,4,5), welches durch Verwendung normierter Bauteile wie Arbeitsaufnahmeplatten (11), Schubladenblöcken (18) für eine oder mehrere Schubkästen (19) oder dergleichen zweckangepaßt konfektionierbar ist, dadurch gekennzeichnet, daß insbesondere die tragenden Bauteile (2,4,5) aus an dem Traggestell befestigbaren Metallteilen bestehen, die sämtlich durch komplementär ausgebildete, die Metallteile umschließende Kunststofformteile (7,9,14) ummantelt sind.

2. Gerätetisch nach Anspruch 1, dadurch gekennzeichnet, daß das Traggestell aus zwei Grundkörper-Seitenteilen (1) aus zwei senkrecht aufragenden Tragsäulen (2)besteht, die lösbar untereinander verbunden sind durch eine gleichzeitig als Handhabe dienende obere Traverse (4) und eine mit Rollen (6) im Bereich ihrer beiden Enden bestückte untere Traverse (5).

3. Gerätetisch nach Anspruch 2, dadurch gekennzeichnet, daß die Tragsäulen (2) mit in bestimmtem Raster angeordneten Aufnahmen (3) für die Befestigung der Bauteile (11,18,22,23,24) versehen sind.

4. Gerätetisch nach Anspruch 2 oder 3, dadurch gekennzeichnet, daß die die Tragsäulen (2) ummantelnden Kunststofformteile (7) im Anschlußbereich an die Traverse (5) von einer an das diese überdeckende Kunststofformteil (7) dichtend anschließenden Manschette (10) umgeben sind.

5. Gerätetisch nach einem der Ansprüche 2 bis 4, dadurch gekennzeichnet, daß das die Traverse (4) überdeckende Kunststofformteil (8) die Endbereiche der Kunststofformteile (7) übergreift und als Handhabe ausgebildet ist.

6. Gerätetisch nach Anspruch 1, dadurch gekennzeichnet, daß jede Arbeitsaufnahmeplatte (11) eine Verstärkungsplatte (12) umfaßt, die an ihrer Unterseite in Längsrichtung durch Traversen (13) versteift ist, wobei die Verstärkungsplatte (12) zwischen einer Arbeitsplatte (16) und einem an die Form der Verstärkungsplatte (12) und die der Traversen (13) angepaßten Kunststofformteil (14 bzw. 15) eingebettet ist.

7. Gerätetisch nach Anspruch 1, dadurch gekennzeichnet, daß sowohl die Schubkästen (19) als auch die diese aufnehmenden Schubladenblöcke (18) aus einem inneren (21) und einem äußeren Kunststofformteil (20) bestehen, zwischen denen Versteifungselemente und/oder Versteifungsplatten eingeordnet sind.

8. Gerätetisch nach Anspruch 7, dadurch gekennzeichnet, daß sowohl die Schubkästen (19) als auch der Schubladenblock (18) aus Kunststoff gefertigt sind.

FIG. 1

FIG. 2

EP 0 401 579 A1

FIG. 3

FIG. 4

FIG. 5

FIG. 6

FIG. 7

FIG. 8

FIG. 9

FIG. 10

EP 0 401 579 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 045 267 (DOLLEY) <br> * Seite 5, Zeile 11 - Seite 9, Zeile 10; Figur 1 * <br> --- | 1 | A 61 B 19/02 <br> A 47 B 31/00 |
| Y | GB-A-1 135 513 (T.W.T.C.) <br> * Seite 1, Zeilen 23-60; Seite 1, Zeile 86 - Seite 2, Zeile 19; Figuren * | 1 | |
| A | | 2,6,7 | |
| | --- | | |
| A | CH-A- 352 112 (MILLION-GUIET-TUBAUTO) <br> * Seite 2, Zeilen 5-21; Figuren * <br> --- | 1,6,7 | |
| A,P | DE-U-8 906 763 (SCHÄFER) <br> * Seite 6, Zeile 12 - Seite 9, Zeile 2; Figuren * <br> --- | 1-3,5 | |
| A | FR-A-1 152 416 (PETIT) <br> * Insgesamt * <br> --- | 2,4 | |
| A | FR-A-1 058 428 (ANGOT) <br> * Seite 1, Spalte 2, Zeile 20 - Seite 2, Spalte 1, Zeile 11; Figuren * <br> --- | 3 | RECHERCHIERTE SACHGEBIETE (Int. Cl.5) |
| A | FR-A-2 550 431 (REY) <br> * Zusammenfassung; Figuren 1,2,4 * <br> --- | 4 | A 61 B <br> A 61 G <br> A 47 B <br> B 62 B |
| A | US-A-3 527 174 (LAY) <br> * Spalte 2, Zeilen 23-30; Figur 3 * <br> --- | 6 | |
| A,P | DE-U-8 905 788 (STOCKER) <br> * Seite 2, Zeilen 1-3; Seite 4, Zeilen 4-7; Seite 4, Zeilen 18-22 * <br> --- | 6,7 | |
| A | US-A-4 241 668 (CARROLL) <br> * Spalte 3, Zeilen 54,55; Figuren * <br> ---      -/- | 8 | |

Der vorliegende Recherchenbericht wurde für alle Patentansprüche erstellt

| Recherchenort | Abschlußdatum der Recherche | Prüfer |
|---|---|---|
| DEN HAAG | 04-09-1990 | KLEIN C. |

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GB-A-2 106 455 (DOHERTY)<br>--- | | |
| A | DE-B-1 056 783 (RAVEN)<br>----- | | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 04-09-1990 | KLEIN C. |